(19) Europäisches Patentamt
European Patent Office
Office européen des brevets

(11) Veröffentlichungsnummer: **0 328 150**
**A2**

(12) # EUROPÄISCHE PATENTANMELDUNG

(21) Anmeldenummer: **89102411.9**

(51) Int. Cl.⁴: **C07D 317/36**

(22) Anmeldetag: **13.02.89**

(30) Priorität: **12.02.88 DE 3804820**

(43) Veröffentlichungstag der Anmeldung:
**16.08.89 Patentblatt 89/33**

(84) Benannte Vertragsstaaten:
**AT BE CH DE ES FR GB GR IT LI LU NL SE**

(71) Anmelder: **DAINIPPON INK & CHEMICALS, INC.**
**7-20, Nihonbashi 3-chome, Chuo-ku**
**Tokyo 100(JP)**

(72) Erfinder: **Grahe, Gerwald, Dr.**
**Breite Strasse 42 b**
**D-1000 Berlin 33(DE)**
Erfinder: **Lachowicz, Artur, Dr.**
**Courbièrestrasse 6**
**D-1000 Berlin 30(DE)**

(74) Vertreter: **Maikowski, Michael, Dipl.-Ing. Dr.**
**Xantener Strasse 10**
**D-1000 Berlin 15(DE)**

(54) **Cyclocarbonathaltige Ester und Verfahren zu ihrer Herstellung.**

(57) Die Erfindung betrifft ein Verfahren zur Herstellung cyclocarbonathaltiger Ester sowie neue Verbindungen.

EP 0 328 150 A2

## Cyclocarbonathaltige Ester und Verfahren zu ihrer Herstellung

Die Erfindung betrifft ein Verfahren zur Herstellung cyclocarbonathaltiger Ester durch Umsetzung cyclocarbonathaltiger Alkohole mit Säureanhydriden sowie neuer Verbindungen.

Es ist bekannt, daß durch Umsetzung epoxidhaltiger Ester mit Kohlendioxid cyclocarbonathaltige Ester erhalten werden können.

Das entsprechende Verfahren wird in der DE-OS 35 29 263 beschrieben. Ähnliche Verfahren beschreiben außerdem G. Rokicki und Mitarbeiter (Monatshefte für Chemie 115 (1984) 205-214). Diese Verfahren zur katalytischen Anlagerung von Kohlendioxid arbeiten in der Regel unter erhöhtem Druck. Weiterhin ist es notwendig, diese epoxidhaltigen Ester in sehr reiner Qualität in das Verfahren einzusetzen. Jedoch können diese Ausgangsverbindungen bisher in den wenigsten Fällen in ausreichender Menge großtechnisch hergestellt werden.

Es ist ebenfalls bekannt, daß man zur Herstellung cyclocarbonathaltiger Ester cyclocarbonathaltige Alkohole, beispielsweise Glycerincyclocarbonat, (V) einsetzen kann.

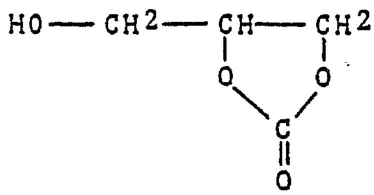

V

Die primäre Alkoholfunktion des Glycerincyclocarbonats wird dabei in Umesterungsprozessen zur Reaktion gebracht.

Auf diese Weise werden insbesondere cyclocarbonathaltige Ester ungesätigter Säuren hergestellt (US-Pat. 2 979 514, US-Pat. 2 967 173).

Auch die Umsetzung von Glycerincyclocarbonat (V) mit Anhydriden ungesättigter Carbonsäuren wurde untersucht. G.F. D'Alelio und T. Huemmer beschreiben im 'Journal of Polymer Science', Part A-1 5 (1967) 307-321 (auch US-Pat. 3 225 063) Versuche zur Synthese cyclocarbonathaltiger Ester aus V und Säureanhydriden.

Zur Erzielung hoher Ausbeuten ist es bei den bekannten Verfahren notwendig, die Reaktion über sehr lange Zeit bei erhöhter Temperatur ablaufen zu lassen, beispielsweise 36-48 h bei 60-80 °C. Unter diesen Bedingungen werden eine Vielzahl von Nebenreaktionen beobachtet, so z. B. die Polymerisation von Acrylsäurederivaten, die Bildung von substituierten Ethylenglykoldiestern (HÜLS AG, Informationsprospekt "Alkylencarbonate", 1985), und Polyestern (GB-Pat. 778 410).

Aus dem Stand der Technik ergibt sich, daß man bei der Synthese cyclocarbonathaltiger Ester aus Säureanhydriden und cyclocarbonathaltigen Alkoholen sehr lange Reaktionszeiten anwenden muß und selbst bei relativ niedrigen Temperaturen die Hauptreaktion oft von Nebenreaktionen begleitet ist.

Gegenstand der vorliegenden Erfindung ist ein Verfahren zur Herstellung cyclocarbonathaltiger Ester der allgemeinen Formel I.

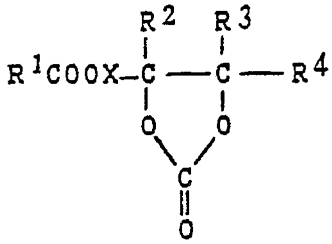

I

worin

$R^1$ ein Wasserstoffatom, einen gesättigten oder ungesättigten, gerad - oder verzweigtkettigen oder cyclischen, aromatischen oder arylaliphatischen, gegebenenfalls substituierten, Kohlenwasserstoffrest mit 1 bis 12 Kohlenstoffatomen oder Etherrest mit 1 bis 12 Kohlenstoffatomen und bis zu 3 Sauerstoffatomen,

$R^2$, $R^3$ und $R^4$ ein Wasserstoffatom oder eine Methylgruppe, die ggf. substituiert sein kann, wobei die Reste

untereinander identisch oder unter schidlich sein können, und X einen zweiwertigen aliphatischen, cycloaliphatischen, aromatischen, arylaliphatischen oder etherhaltigen, ggf. substituierten Kohlenwasserstoffrest mit 1 bis 20 Kohlenstoffatome bedeuten, dadurch gekennzeichnet, daß ein cyclocarbonathaltiger Alkohol der allgemeinen Formel II,

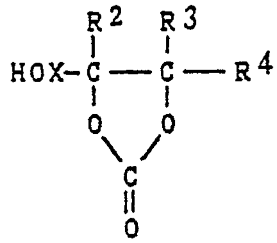

II

wobei X, $R^2$, $R^3$ und $R^4$ die vorstehend angegebene Bedeutung haben, mit einem Carbonsäureanhydrid der allgemeinen Formel III,

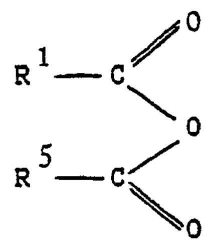

III

wobei $R^1$ die vorstehend angegebene Bedeutung hat und $R^5$ identisch mit $R^1$ sein kann oder $R^1$ und $R^5$ Bestandteile eines Ringes sein können, bei erhöhten Temperaturen und Reaktionszeiten bis zu sechs Stunden umgesetzt werden.

Als mögliche Reste von $R^1$ der allgemeinen Formel I sind ein Wasserstoffatom oder gesättigte oder ungesättigte, gerad- oder verzweigtkettige, cyclische, aromatische oder arylaliphatische, ggf. substituierte, Kohlenwasserstoffreste mit 1 - 12 Kohlenstoffatomen oder Etherreste mit 1 - 12 Kohlenstoffatomen und bis zu 3 Sauerstoffatomen zu nennen. Als besonders gut geeignet sind Methyl-, Ethyl-, Benzyl-, Phenyl-, Methoxymethyl-, Chlormethyl-, 4-Chlorphenyl-, 3-Chlorphenyl-, 2-Chlorphenyl-, 1-Methyl-vinyl-, Allyl-, 2-Carbonsäureethyl-, 2-Carbonsäurevinyl- und 2-Phenylethylreste zu nennen.

Die möglichen Reste $R^2$, $R^3$ und $R^4$ können untereinander identisch oder verschieden sein. $R^2$, $R^3$ und $R^4$ können jeweils ein Wasserstoffatom oder eine substituierte Methylgruppe bedeuten. Als mögliche Substituenten der Methylgruppe finden Halogene, Alkoxygruppen und aromati sche Reste Anwendung. Besonders zu nennen sind Fluor-, Chlor-, Brom-, Jod-, Methoxy-, Ethoxy- und Phenylreste.

Der zweiwertige Rest X hat die Bedeutung eines aliphatischen, aromatischen, arylaliphatischen oder etherhaltigen, ggf. substituierten Kohlenwasserstoffrestes mit 1 - 20 Kohlenstoffatomen. Gut geeignet sind die Methylen-, 1,2 Ethylen-, 1,3 Propylen und 1,4 Butylenreste. Aber auch Reste, die beispielsweise Ether-, Ester und Urethangruppen enthalten sind besonders geeignet.

Die im erfindungsgemäßen Verfahren einzusetzenden Carbonsäurenanhydride III können eine sehr vielfältige Struktur aufweisen. Es kann sich dabei um Anhydride gesättigter aliphatischer Carbonsäuren (z. B. Acetanhydrid, Propionanhydrid), Anhydride aromatischer Säuren (z. B. Benzoylanhydrid) oder Verbindungen, die im Rest R1 verschiedene Substituenten aufweisen (z. B. Chloracetanhydrid, Methoxyessigsäureanhydrid, 4-Chlorbenzoylanhydrid, Phenylessigsäureanhydrid) handeln. Weitere Gruppen von Anhydridkomponenten III, die erfindungsgemäß eingesetzt werden können, bilden Anhydride ethylenisch ungesättigter Monocarbonsäuren, wie Acryl-, Methacryl-, Croton- oder Zimtsäure.

Polybasische Carbonsäuren und deren Derivate können ebenfalls vorteilhaft bei dem vorliegenden erfindungsgemäßen Verfahren eingesetzt werden. Dies betrifft insbesondere die cyclischen Anhydride von Dicarbonsäuren, wie Malein-, Bernstein-, Itacon-, Trimellit- oder Pyromellitsäureanhydrid.

Eine weitere Gruppe von Säureanhydriden, die gemäß der vorliegenden Erfindung mit cyclocarbonathaltigen Alkoholen umgesetzt werden kann, bilden Polymere mit Anhydridgruppen. Diese werden erhalten aus ethylenisch ungesättigten Carbonsäurenanhydriden, z. B. Malein-, Itacon- bzw. Methacrylanhydrid, durch Polymerisation bzw. vorteilhaft Copolymerisation mit anderen geeigneten Vinyl- bzw. Acrylmonomeren. Die dabei resultierenden Polymerisate besitzen unveränderte Anhydridgruppierungen, die gemäß der vorliegenden Erfindung an der Reaktion teilnehmen können. Höhermolekulare, polyfunktionelle Carbonsäureanhydride, die vorteilhaft nach dem erfindungsgemäßen Verfahren eingesetzt werden können, lassen sich ebenfalls durch Polykondensations- bzw. Polyadditionsreaktionen herstellen, z. B. durch entsprechende Umsetzung von Pyromellitsäureanhydrid mit Polyolen, bzw. Trimellitsäureanhydrid mit Polyisocyanaten.

Das erfindungsgemäße Verfahren kann mit und ohne Lösungsmittel durchgeführt werden. Im Allgemeinen wird jedoch die Druchführung der Umsetzung in Gegenwart eines Lösungsmittels oder eines Lösungsmittelsgemisches bevorzugt. Als Lösungsmittel sind aromatische oder nichtaromatische Kohlenwasserstoffe, die auch mit Halogenen substituiert sein können, geeignet. Aber auch ester-, ether-, carbonsäureamid-, sulfon- und sulfoxidhaltige Lösungsmittel sind zur Reaktionsdurchführung anwendbar. Als besonders geeignet ist Toluol, Xylol, Chlorbenzol, Dichlorbenzol, Brombenzol, Essigsäurebutylester, Hexamethylphosphorsäuretriamid, 2,6-Di-tert. -butyl-4-methylphenol, Dimethylsulfoxid und Dimethylformamid, Dimethoxyethan, Ethylendiglykolacetat und Dimethylacetamid zu nennen.

Aber auch Lösungsmittelgemische bestehend aus zwei oder mehreren dieser Lösungsmittel sind anwendbar.

Für die Reaktionsdurchführung ist es nötig, daß die Reaktionspartner der allgemeinen Formeln II und III im Molverhältnis II:III von 3:1 bis 1:3, vorzugsweise 1, 5:1 bis 1:1, 5, insbesonders 1, 2:1 bis 1:1, 2, ungesetzt werden.

Die Reaktion wird durch Zugabe von Katalysatoren, sauren oder basischen beschleunigt. Als Katalysatoren sind Protonensäuren, wie z. B. Schwefelsäure, p-Toluolsulfonsäure, Chlorwasserstoff und Phosphorsäure oder Lewis-Säuren, wie z. B. Zinkchlorid, Titantetrachlorid und Zinntetrachlorid oder Basen wie z. B. Triethylamin und Pyridin geeignet. Die Reaktion wird bei Temperaturen von 60 bis 140 °C, vorzugsweise von 80 - 120 °C, durchgeführt.

In Anbetracht des Standes der Technik muß es als sehr überraschend bezeichnet werden, daß das erfindungsgemäße Verfahren die Herstellung cyclocarbonathaltiger Ester in hohen Ausbeuten bei gleichzeitig kurzen Reaktionszeiten gestattet.. Die Synthese cyclocarbonathaltiger Ester nach der vorliegenden Erfindung ist nach ca. 4 h abgeschlossen , während die bekannten Verfahren ca. 10fach längere Reaktionszeiten beanspruchen (US-Pat. 3 225 065). Es ist darüberhinaus äußerst überraschend, daß auch bei erhöhten Prozesstemperaturen - im Gegensatz zu bekannten Verfahren (GB-Pat. 778 410) - keine Reaktion zwischen dem Cyclocarbonatring und den Säureanhydriden zu beobachten ist. Auch andere Nebenreaktionen wurden nicht bemerkt.

Das erfindungsgemäße Verfahren bedeutet daher einen erheblichen Vorteil gegenüber den bekannten Verfahren. Die nach dem erfindungsgemäßen Verfahren erhaltenen cyclocarbonathaltigen Ester können in verschiedenen Bereichen der chemischen oder pharmazeutischen Industrie eingesetzt werden.

Eine Gruppe cyclocarbonathaltiger Alkohole, die bei dem erfindungsgemäßen Verfahren mit Carbonsäureanhydriden ebenfalls vorteilhaft ungesetzt werden kann, bilden Cyclocarbonatgruppen enthaltende Oligo- bzw. Polymere, die zusätzlich mindestens eine Hydroxylfunktion besitzen, z. B. cyclocarbonatmodifizierte Epoxidharze der allgemeinen Formel IV,

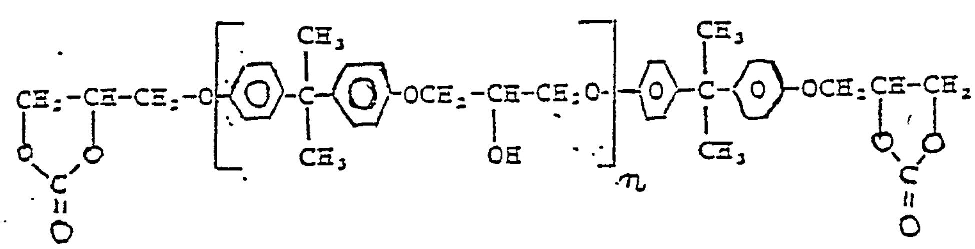
IV.

die aus den handelsüblichen Epoxidharzen und CO2, beispielsweise nach DE-OS 35 29 263 bzw. DE-OS 36 00 602, erhalten werden können. Die Harze VI können an den vorhandenen sekundären Hydroxylgruppen nach dem erfindunsgemäßen Verfahren mit Carbonsäureanhydriden verestert werden.

Analoge Reaktionen lassen sich nach dem erfindungsgemäßen Verfahren mit zahlreichen weiteren hydrodxylfuntionellen Oligo- bzw. Polymeren ausführen, die Cyclocarbonatgruppen besitzen, beispielsweise mit modifizierten Polyester- bzw. Polyetheralkoholen,

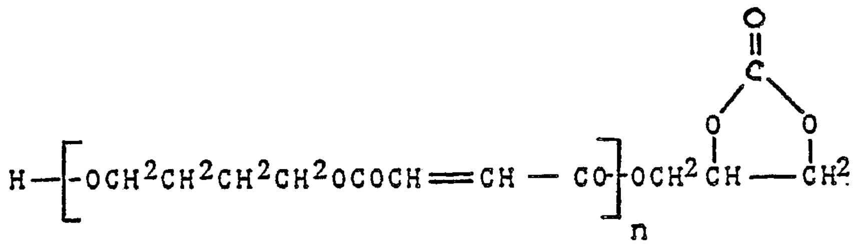

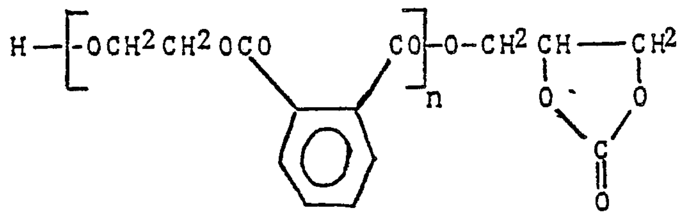

und mit modifizierten Polyurethanen

- 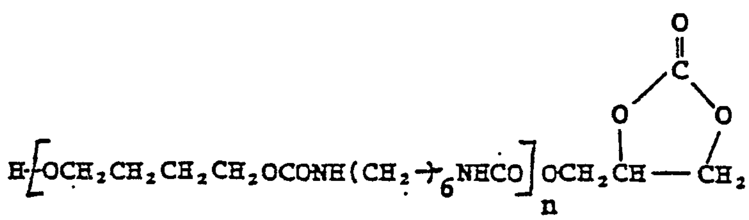

- 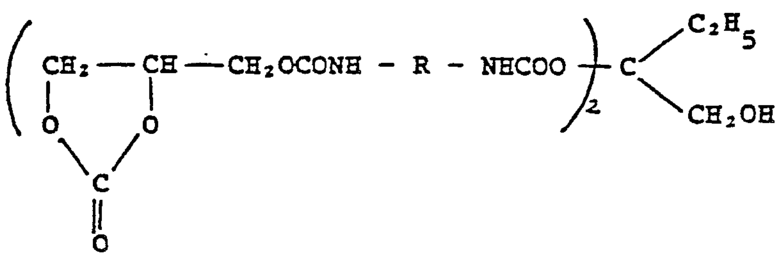

- 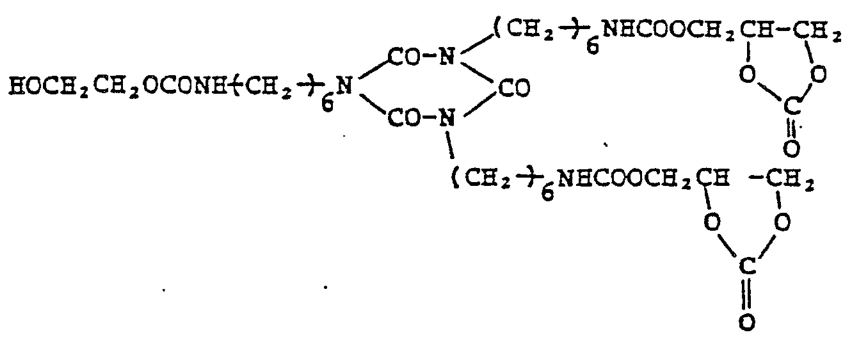

Besonders interessant ist die Verwendung der nach dem erfindungsgemäßen Verfahren erhaltenen cyclocarbonathaltigen Ester zur Herstellung von Urethanen. Die entsprechende Reaktion mit primären bzw. sekundären Aminen verläuft gemäß der Gleichung

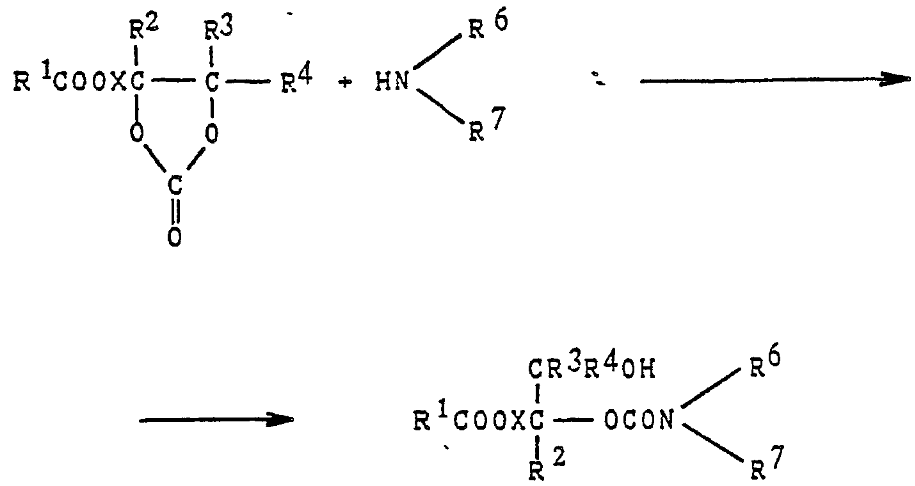

Die Reste $R^6$ und $R^7$ haben die Bedeutung eines Wasserstoffatoms oder eines aliphatischen, cycloaliphatischen oder aromatischen, gegebenfalls substituierten Kohlenwasserstoffrestes mit 1 bis 12 Kohlenstoffatomen.

Setzt man bei der Reaktion polyfunktionelle cyclocarbonathaltige Ester mit polyfunktionellen Aminen um, so kann man Polyurethane erhalten, die überlicherweise aus den hochgiftigen Isocyanaten hergestellt werden. Besonders interessant ist daher die Herstellung cyclocarbonathaltiger Ester nach dem erfindungsgemäßen Verfahren, die zumindest eine polymerisierbare Funktion besitzen. Es handelt sich hierbei beispielsweise um

a) cyclocarbonathaltige Ester monobasischer Carbonsäuren, wie Acryl-, Methacryl- bzw. Crotonsäure, z. B.

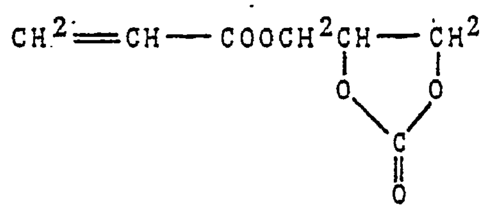

b) cyclocarbonathaltige Ester di- oder polybasischer Säuren, wie Malein-, Fumar- bzw. Itaconsäure, z. B.

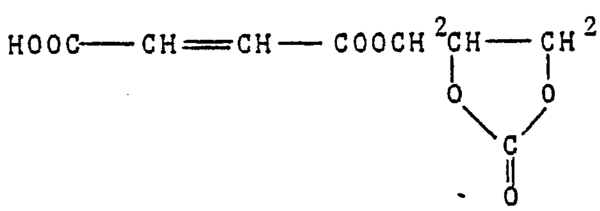

Solche Substanzen können durch Polymerisation in Polymere mit unveränderten Cyclocarbonatfunktionen überführt werden, was am Beispiel des cyclocarbonathaltigen Esters der Methacrylsäure gezeigt wird,

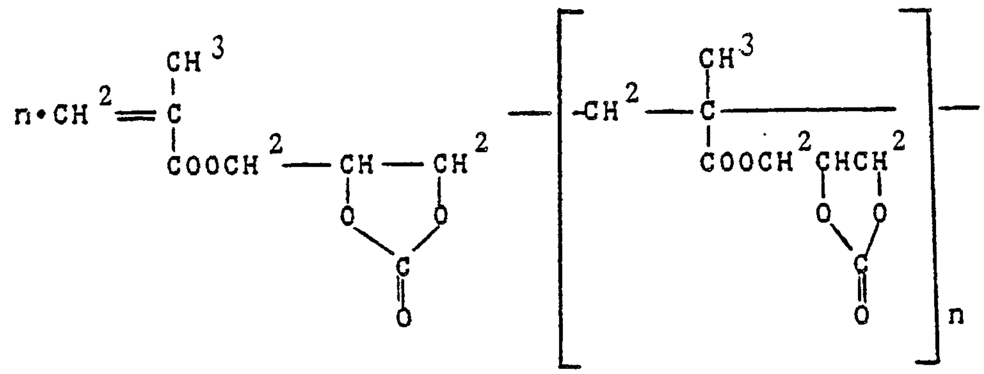

Solche linearen Polymere sind in organischen Lösungsmitteln löslich. Durch die Umsetzung mit Di- oder Polyaminen können sie in eine Netzwerkstruktur übergehen.

Auf diese Weise entstehen dreidimensionale Netzwerke, die Polyurethanbrücken aufweisen. Solche System, die gewöhnlich durch Reaktion hydroxylhaltiger Polymere mit Isocyanaten erhalten werden, spielen beispielsweise auf dem Beschichtungssektor eine außerordentlich wichtige Rolle.

Mit dem Einsatz der nach erfindungsgemäßen Verfahren hergestellten polymerisierbaren cyclocarbonathaltigen Ester lassen sich Polyurethanstrukturen ohne Verwendung von Isocyanaten herstellen.

Zu diesem Zweck kann man sowohl Homopolymere der einzelnen cyclocarbonathaltigen Estermonomeren

z.B. 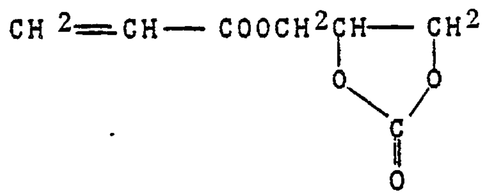

als auch ihre Copolymere miteinander oder in Kombinationen mit einem oder mehreren üblichen Vinyl-oder Acrylmonomeren bzw. vergleichbaren Verbindungen (z. B. Maleinsäure oder - ester) einsetzen.

Die Polymere mit Cyclocarbonatfunktionen ergeben nach Vermischen mit Di- oder Polyaminen Gemische, die zu klaren oder pigmentierten Filmen, die glänzende, harte und lösungsmittelbeständige Eigenschaften haben, ausgehärtet werden können, die sämtliche Vorteile einer gewöhnlichen Polyurethanbeschichtung aufweisen.

Die folgenden Beispiele sollen das erfindungsgemäße Verfahren näher erläutern:

Beispiel 1

Ein Gemisch aus 102 g Essigsäureanhydrid, 118 g Glycerincyclocarbonat und 0.5 g p-Toluolsulfonsäure wird 1 Stunde bei 100-105 °C gerührt und einer Vakuumdestillation unterworfen. Nach einem Vorlauf destilliert 4-(2-Oxo-1,3-dioxolanyl)-methyl-acetat (Glycerincyclocarbonatacetat) als farblose Flüssigkeit.
Kp: 108 ° C / 0,01 mbar
Ausbeute: 155 g (ca. 97% d. Th.)

Beispiel 2

Ein Gemisch aus 102 g Essigsäureanhydrid, 40 g Glycerincyclocarbonat und 5 g Pyridin wird 2 Stunden bei 100-105 °C gerührt. Die Aufarbeitung erfolgt analog Beispiel 1
Ausbeute an Glycerincyclocarbonatacetat: 47 g (ca. 87% d. Th.)
Kp: 107-109 ° C / 0,01 mbar.

Beispiel 3

Ein Gemisch aus 35 g Essigsäureanhydrid, 40 g Glycerincyclocarbonat, 0.5 g Phosphorsäure (85%ig in Wasser) und 100 g Toluol wird bei 110° C 1 Stunde gerührt. Die Auf arbeitung erfolgt analog Beispiel 1.
Ausbeute an Glycerincyclocarbonatacetat: 46 g (ca. 85% d. Th.)
Kp: 106-108° C / 0.01 mbar.

Beispiel 4

Ein Gemisch aus 50 g Essigsäureanhydrid, 40 g Glycerincyclocarbonat, 0,5 g Zinkchlorid und 100 g Xylol wird 2 Stunden bei 110° C gerührt. Die Aufarbeitung erfolgt analog Beispiel 1.
Ausbeute an Glycerincyclocarbonatacetat: 50.5 g (ca. 93% d. Th.)
Kp: 109-110° C / 0.01 mbar.

Beispiel 5

Ein Gemisch aus 35 g Propionsäureanhydrid, 29 g Glycerincyclocarbonat, 0,5 g konz. Schwefelsäure und 50 g Chlorbenzol wird 2 Stunden bei 100-105° C gerührt. Die Aufarbeitung erfolgt durch Vakuumdestillation analog Beispiel 1. Das 4-(2-Oxo-1,3-dioxolanyl)-methylpropionat (Glycerincyclocarbonatpropionat) destilliert als farblose, leichtbewegliche Flüssigkeit bei 126-128° C / 0.01 mbar.
Ausbeute: 39 g (ca. 91 % d. Th.)

Beispiel 6

Ein Gemisch aus 70 g Propionsäureanhydrid, 29 g Glycerincyclocarbonat, 2 g Triethylamin und 100 ml Dimethoxyethan wird 3 Stunden unter Rückfluß erhitzt. Die Aufarbeitung erfolgt analog Beispiel 1.
Ausbeute an Glycerincyclocarbonatpropionat: 36 g (ca. 84% d. Th.)
Kp: 125-126° C / 0.01 mbar.

Beispiel 7

Ein Gemisch aus 29 g Glycerincyclocarbonat, 80 g Isobuttersäureanhydrid und 100 g Dimethylsulfoxid wird 2 Stunden bei 100-105° C gerührt. Anschließend wird das Gemisch durch fraktionierende Vakuumdestillation aufgearbeitet. Nach einem Vorlauf destilliert Glycerincyclocarbonatisobutyrat als klare farblose Flüssigkeit bei 133-135° C / 0.01 mbar.
Ausbeute: 40,5 g (ca. 87,5% d. Th.)

Beispiel 8

Ein Gemisch aus 29 g Glycerincyclocarbonat, 60 g Chloressigsäureanhydrid und 50 g Dimethylformamid wird 2 Stunden bei 100° C gerührt. Bei der anschließenden fraktionierenden Vakuumdestillation geht das 4-(2-Oxo-1,3-dioxolanyl)-methyl-chloracetat (Glycerincyclocarbonatchloracetat) als hellgelbe Flüssigkeit bei 135-138° C/ 0.01 mbar über. Beim Abkühlen erstarrt das Destillat zu farblosen Kristallen.
Ausbeute: 31 g (ca. 65% d. Th.)

Beispiel 9

Ein Gemisch aus 60 g Methoxyessigsäureanhydrid, 29 g Glycerincyclocarbonat und 100 g Butylacetat wird 2 Stunden bei 100-105 C gerührt. die Aufarbeitung erfolgt durch fraktionierende Vakuumdestillation. Das 4-(2-Oxo-1,3-dioxolanyl)-methyl-methoxyacetat (Glycerincyclocarbonatmethoxyaceat) geht als farblose, viskose Flüssigkeit bei 130-135° C / 0.01 mbar über.
Ausbeute: 36 g (ca. 77% d. Th.)

Beispiel 10

Ein Gemisch aus 50 g Phenylessigsäureanhydrid, 35 g Glycerincyclocarbonat, 0,5 g p-Toluolsulfonsäure und 150 g Toluol wird 2 Stunden am Rückfluß erhitzt. Anschließend wird das Gemisch abgekühlt und mit einer Lösung aus 50 g Natriumhydrogencarbonat in 50 ml Wasser sowie mit 200 ml Wasser gewaschen. Die organische Schicht wird abgetrennt und im Vakuum bei ca. 100° C eingeengt. Der resultierende Rückstand wird dann im Hochvakuum destilliert. Bei 180-185° C / 0.001 mbar destilliert das Glycerincyclocarbonatphenylacetat als eine gelbliche, viskose Flüssigkeit.
Ausbeute: 26.5 g (ca. 57% d. Th.)

Beispiel 11

Ein Gemisch aus 45 g Benzoesäureanhydrid, 35 g Glycerincyclocarbonat, 0,5 g p-Toluolsulfonsäure und 150 g Toluol wird 2 Stunden am Rückfluß erhitzt. Die Aufarbeitung erfolgt analog Beispiel 10. Das Glycerincyclo carbonatbenzoat geht bei 172-176° .C / 0.001 mbar als farblose, viskose Flüssigkeit über.
Ausbeute: 25.5 g (ca. 58% d. Th.)

Beispiel 12

Ein Gemisch 50 g 3-methylbenzoesäureanhydrid, 35 g Glycerincyclocarbonat, 0,5 g p-Toluolsulfonsäure und 150 g Toluol wird analog Beispiel 10 zur Reaktion gebracht und aufgearbeitet. Das Glycerincyclocarbonat-3-methylbenzoat destilliert bei 178-182° C / 0.001 mbar als eine gelbliche, viskose Flüssigkeit.
Ausbeute: 22 g (ca. 47% d. Th.)

Beispiel 13

Ein Gemisch aus 50 g Crotonsäureanhydrid, 58 g Glycerincyclocarbonat, 0,5 g p-Toluolsulfonsäure, 0,5 g 2,6-Ditert.-butyl-4-methylphenol und 150 g Xylol wird 1 Stunde am Rückfluß erhitzt. Das abgekühlte Reaktionsgemisch wird, analog Beispiel 10, aufgearbeitet. Das 4-(2-Oxo-1,3-dioxolanyl)-methyl-crotonat (Glycerincyclocarbonatcrotonat) distilliert bei 130-132° C / 0.01 mbar als farblose Flüssigkeit.
Ausbeute: 52.5 g (ca. 87% d. Th.)

Beispiel 14

Ein Gemisch aus 500 g Methacrylsäureanhydrid, 450 g Glycerincyclocarbonat, 1 g p-Toluolsulfonsäure und 2 g 2,6-Di-tert-butyl-4-methylphenol wird 4 Stunden bei 85- 90° C gerührt. Das resultierende Reaktionsgemisch wird anschließend einer kontinuierlich betriebenen Kurzwegdestillation unterworfen. Bei ca. 120° C / 0.01 mbar siedet das Glycerincyclocarbonatmethacrylat als eine farblose, etwas viskose Flüssigkeit (Reinheit: 98-99% nach GC).
Ausbeute: 524 g (ca. 87% d. Th.)

Beispiel 15

Ein Gemisch aus 30 g Maleinsäureanhydrid, 29 g Glycerincyclocarbonat, 0,5 g p-Toluolsulfonsäure und 150 g Toluol wird 3 Stunden bei 105-110° C gerührt. Das Glycerincyclocarbonatmonomaleinat kristallisiert beim Abkühlen in Form von farblosen Kristallen, Fp: 107-110° C.
Ausbeute: 44 g (ca. 83% d. Th.)

Beispiel 16

Ein Gemisch aus 32 g Itaconsäureanhydrid, 29 g Glycerincyclocarbonat, 0,5 g p-Toluolsulfonsäure, 0,5 g 2,6-Ditert-butyl-4-methylphenol und 150 g Toluol wird 4 Stunden bei 90-95° C gerührt. Das Glycerincyclocarbonatmonoitaconat kristallisiert beim Abkühlen in Form von farblosen Kristallen, Fp: 127-130° C.

Ausbeute: 42 g (ca. 74% d. Th.)

Beispiel 17

Ein Gemisch aus 33 g 4-(2-Hydroxyethyl)-2-oxo-1,3-dioxolan, 40 g Essigsäureanhydrid, 0,5 g p-Toluolsulfonsäure und 100 g Toluol wird 2 Stunden bei 100°C gerührt. Anschließend wird das Gemisch durch fraktionierende Vakuumdestillation aufgearbeitet. Das 4-(2-Oxo-1,3-dioxolanyl)-ethyl-acetat siedet bei 132-135° C / 0.001 mbar als farblose Flüssigkeit.
Ausbeute: 35 g (ca. 80.5% d. Th.)

Beispiel 18

Zu einer Lösung von 126 g Hexamethylendiisocyanat in 200 ml Dimethoxyethan wird innerhalb 1 Stunde bei Raumtemperatur 29 g Glycerincyclocarbonat eingetropft. Das Gemisch wird anschließend 1 Stunde bei 60°C gerührt. In einer kontinuierlich betriebenen Kurzwegdestillationsapparatur wird dann bei 120° C / 0.01 mbar das Lösungsmittel und das überschüssige Diisocynat entfernt. Der Destillationsrückstand wird dann mit 80 g Ethylenglykol und 100 g Dimethoxyethan vermischt und bei 160° C 1 Stunde gerührt. Das Lösungsmittel und das überschüssige Ethylenglykol wird anschließend im Vakuum bei 120° C / 0.01 mbar abdestilliert und der Destillationsrückstand wird mit 100 g Essigsäureanhydrid vermischt und bei 100 C 2 Stunden gerührt. Die entstandene Essigsäure und das überschüssige Essigsäureanhydrid wird bei 120° C / 0.01 mbar abdestilliert. Das erhaltene 1-(4-(2-Oxo-1,3-dioxolanyl)-methyloxycarbonylamino-6-((2-acetoxy)-ethyl-oxycarbonylamino)-hexan fällt als Destillationsrückstand in Form eines gelblichen Öles an.
Ausbeute: 93 g (ca. 97% d. Th.)
Das Reaktionsprodukt enthält keine freien Isocyanat-bzw. Hydroxylgruppen (nach IR-Analyse).
IR-Absorption: 1795 cm -1.

Beispiel 19

Epiclon 1050, ein Glycidylether des Bisphenol A (Hersteller: "Dainippon Ink & Chemicals, Inc., Japan) mit folgender Struktur:

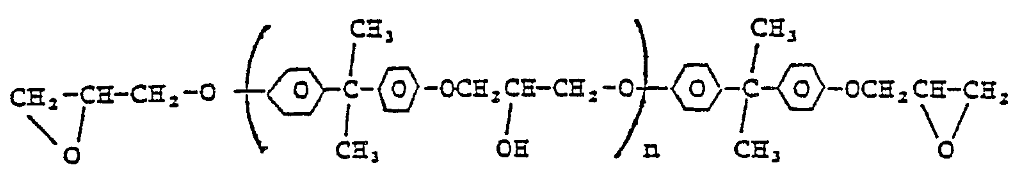

n = 2,1

wird analog DE-OS 35 29 263 in eine cyclocarbonathaltige Verbindung überführt und nach folgender Vorschrift verbessert.

100 g des lösungsmittelfreien Produktes (Hydroxylzahl: 120-130 mg KOH/g) wird mit 60 g Methacrylsäureanhydrid, 0,5 g 2,6-Di-tert.-butyl-4-methylphenol und 0,5 g p-Toluolsulfonsäure vermischt und 6 Stunden bei 80°C gerührt. Anschließend wird das Reaktionsgemisch in einer kontinuierlich betriebenen Kurzwegdestillationsapparatur bei 100° C / 0.01 mbar von den flüchtigen Bestand teilen befreit. Der als gelbbraunes Öl anfallende Destillationsrückstand besteht aus einem mehrfach funktionellen Ester der Methacrylsäure mit folgender Struktur:

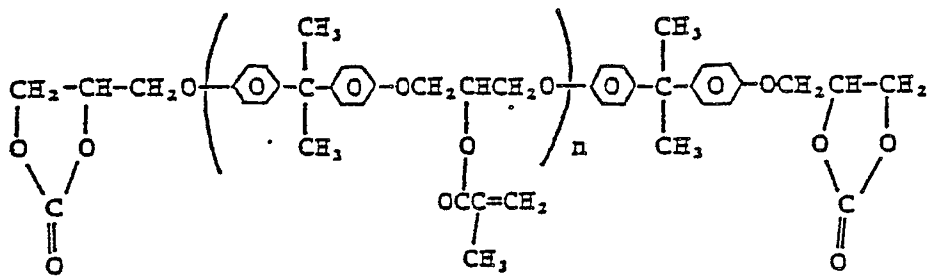

n = 2,1

Ausbeute: 110 g
Das Produkt enthält keine Epoxidgruppen.
Hydroxylzahl: 8-10 mg KOH/g; IR-Absorption: 1795 cm-1.

## Ansprüche

1. Verfahren zur Herstellung cyclocarbonathaltiger Ester der allgemeinen Formel I,

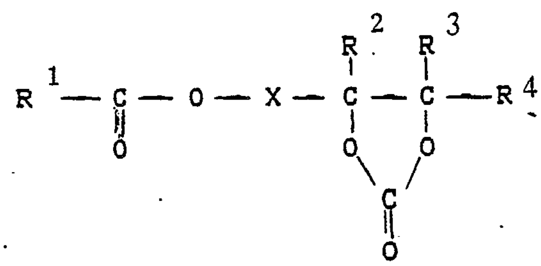

I

worin
$R^1$ ein Wasserstoffatom, einen gesättigten oder ungesättigten, gerad- oder verzweigtkettigen oder cyclischen, aromatischen oder arylaliphatischen substituierten oder unsubstituierten Kohlenwasserstoffrest mit 1 bis 12 Kohlenstoffatomen oder Etherrest mit 1 bis 12 Kohlenstoffatomen und bis zu 3 Sauerstoffatomen,
$R^2$, $R^3$ und $R^4$ ein Wasserstoffatom oder eine Methylgruppe, die ggf. substituiert sein kann, wobei die Reste untereinander identisch oder unterschiedlich sein können, und
X einen zweiwertigen aliphatischen, cycloaliphatischen, aromatischen, arylaliphatischen oder etherhaltigen, ggf. substituierten Kohlenwasserstoffrest mit 1 bis 20 Kohlenstoffatomen
bedeuten,
dadurch gekennzeichnet, daß
ein cyclocarbonathaltiger Alkohol der allgemeinen Formel II,

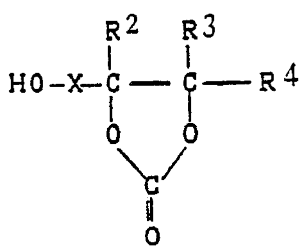

II

wobei X, $R^2$, $R^3$ und $R^4$ die vorstehend angegebene Bedeutung haben,
mit einem Carbonsäureanhydrid der allgemeinen Formel III,

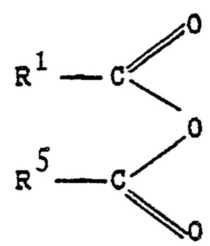

III

wobei
$R^1$ die vorstehend angegebene Bedeutung hat und R5 identisch mit $R^1$ ist oder $R^1$ und $R^5$ Bestandteile eines Ringes sind,
bei erhöhten Temperaturen und Reaktionszeiten bis zu sechs Stunden umgesetzt wird.

2. Verfahren nach Anspruch 1,
dadurch gekennzeichnet, daß
die Herstellung der cyclocarbonathaltigen Ester in Gegenwart eines Lösungsmittels duchgeführt wird.

3. Verfahren nach Anspruch 2,
dadurch gekennzeichnet, daß
als Lösungsmittel aliphatische oder aromatische, ggf. halogensubstituierte Kohlenwasserstoffe, Ester, Ether, Glykoletherester, Amide, Sulfoxide oder deren Gemische eingesetzt werden.

4. Verfahren nach mindestens einen der Ansprüche 1 bis 3,
dadurch gekennzeichnet, daß
die Herstellung der cyclocarbonathaltiger Ester in Gegenwart eines Katalysators durchgeführt wird.

5. Verfahren nach Anspruch 4,
dadurch gekennzeichnet, daß
als Katalysator saure oder basische Stoffe eingesetzt werden.

6. Verfahren nach Anspruch 5,
dadurch gekennzeichnet, daß
als Katalysator Protonensäuren, wie Schwefelsäure, p-Toluolsulfonsäure, Chlorwasserstoff und Phosphorsäure oder Lewis-Säuren, wie Zinkchlorid, Titantetrachlorid und Zinntetrachlorid oder Basen, wie Triethylamin und Pyridin eingesetzt werden.

7. Verfahren nach mindestens einem der Ansprüche 4 bis 6,
dadurch gekennzeichnet, daß
die Herstellung der cyclocarbonathaltigen Ester bei Temperaturen zwischen 60 °C bis 150 °C, vorzugsweise zwischen 80 °C bis 120 °C durchgeführt wird.

8. Verfahren nach mindestens einem der Ansprüche 1 bis 7,
dadurch gekennzeichnet, daß
die cyclocarbonathaltigen Alkohole mit den Carbonsäureanhydriden im Molverhältnis von 3:1 bis 1:3, vorzugsweise 1, 5:1 bis 1:1, 5, insbesondere 1, 2:1 bis 1:1, 2, umgesetzt werden.

9. Cyclocarbonathaltige Ester:
4-(2-Oxo-1,3-dioxolanyl)-methyl-propionat 4-(2-Oxo-1,3-dioxolanyl)-methyl-chloracetat
4-(2-Oxo-1,3-dioxolanyl)-methyl-methoxyacetat
4-(2-Oxo-1,3-dioxolanyl)-methyl-crotonat
2-(4-Oxo-1,3-dioxolanyl))-ethyl-acetat
1-(4-(2-Oxo-1,3-dioxolanyl)-methyl-oxycarbonylamino)-6-((2-acetoxy)-ethyl-oxycarbonylamino)-hexan